Europäisches Patentamt

European Patent Office

Office européen des brevets

(19)

(11) Veröffentlichungsnummer: **0 023 989**
**B1**

(12) **EUROPÄISCHE PATENTSCHRIFT**

(45) Veröffentlichungstag der Patentschrift: **28.12.83**

(21) Anmeldenummer: **80104220.1**

(22) Anmeldetag: **18.07.80**

(51) Int. Cl.³: **G 01 N 33/54**

(54) **Enzym-Immuntest zum Nachweis von Erreger-spezifischen Antikörpern und Testsatz für die Durchführung dieses Tests.**

(30) Priorität: **28.07.79 DE 2930706**

(43) Veröffentlichungstag der Anmeldung:
**18.02.81 Patentblatt 81/7**

(45) Bekanntmachung des Hinweises auf die Patenterteilung:
**28.12.83 Patentblatt 83/52**

(84) Benannte Vertragsstaaten:
**AT BE CH DE FR GB IT LI NL SE**

(56) Entgegenhaltungen:
**EP - A - 0 008 473**
**EP - A - 0 009 198**
**DE - A - 2 733 380**
**FR - A - 2 367 286**
**FR - A - 2 433 749**
**US - A - 3 839 153**
**US - A - 4 021 534**
**US - E - 29 169**

Die Akte enthält technische Angaben, die nach
dem Eingang der Anmeldung eingereicht wurden
und die nicht in dieser Patentschrift enthalten
sind.

(73) Patentinhaber: **Medac Gesellschaft für klinische Spezialpräparate mbH**
**Fehlandtstrasse 3**
**D-2000 Hamburg 36 (DE)**

(72) Erfinder: **Schmitz, Herbert, Dr. Prof.**
**Parchimerstrasse 55**
**D-2000 Hamburg 73 (DE)**

(74) Vertreter: **UEXKÜLL & STOLBERG Patentanwälte**
**Beselerstrasse 4**
**D-2000 Hamburg 52 (DE)**

(56) Entgegenhaltungen:
THE JOURNAL OF IMMUNOLOGY, Band 116, Nr. 6, Juni 6. 1976, The Williams & Wilkins CO Baltimore US K. KATO et al.: "Enzyme-linked immunoassay: conjugation of the FAB' fragment of rabbit IG with beta-d-galactosidase from gE. Coli and its use for immunoassay", Seiten 1554-1560
ARZNEIMITTEL FORSCHUNG, Band 28, Nr. 11A, November 1978, Seiten 1934-1940 Aulendorf DE L. SCHROTT: "Enzyminnunoassey"

## Enzym-Immuntest zum Nachweis von Erreger-spezifischen Antikörpern und Testsatz für die Durchführung dieses Tests

Die Erfindung betrifft einen Enzymimmuntest zum Nachweis von Antikörpern, die gegen bestimmte Erreger gerichtet sind, und einen Testsatz für die Durchführung dieses Tests. Ein solcher Test ist für die Diagnostik von großer Bedeutung. Dies gilt insbeseondere für IgM- und IgA-Antikörper, die nach einer akuten Infektion nur für kurze Zeit im Serum von Patienten nachweisbar sind. Durch den Nachweis spezifischer IgM- oder IgA-Antikörper in Serumproben lassen sich akute Infektionen diagnostizieren.

Der Nachweis von gegen spezifische Erreger gebildeten Antikörpern ist bekannt. Er erfolgt bei den bekannten Immunofluoreszenz-, Radioimmun- oder Enzymimmuntests in der Weise, daß man die Antikörper an Antigen bindet und die erfolgte Bindungsreaktion mit markiertem Antikörper feststellt. Diese Verfahrensweise ist jedoch mit einer Reihe von Fehlern behaftet, die häufig zu falschen Befunden führen. Eine besondere Rolle spielen hierbei die Rheumafaktoren, die mit aggregiertem IgG reagieren. Hiebei kommt es zu einer Bindung von Rheumafaktor-IgM, dessen Nachweis z.B. mit markiertem Anti-IgM zu falsch positiven Reaktionen führt, selbst wenn im Serum nur geringe Mengen Rheumafaktor enthalten sind. So wird bezüglich der von Nakane und Mitarbeitern in J. Histochem. Cytochem., Bd. 22 (1974), S. 1084—1091 beschriebenen Peroxidasemarkierung von IgG von G. B. Wisdeom in Clinical Chemistry, Bd. 22, Heft 8, S. 1243 (1976) ausgeführt, daß die Peroxidase mehrere Polysaccharid-moleküle aufweist und nach ihrer Aktivierung durch Einführung von Aldehydgruppen dazu neigt, IgG Moluküle zu Konjugaten mit hohem Molekulargewicht zu vernetzen, zu denen Rheumafaktoren hohe Affinität besitzen.

Falsch positive Befunde werden auch durch antinukleäre Antikörper hervorgerufen, da die meisten im Handel erhältlichen Antigene Kernmaterial enthalten. Schließlich enthalten viele virale Antigene $F_c$-Receptoren, die ebenfalls zu unspezifischen Bindungen führen können. Zu beachten ist ferner, daß insbesondere beim Neugeborenen, das hohe mütterliche IgG-Antikörperkonzentrationen aufweist, die Bindung spezifischer IgM-Antikörper am Antigen durch IgG-Antikörper blockiert werden kann. Falsche Befunde werden auch dadurch verursacht, daß die beim bekannten Nachweisverfahren verwendeten markierten Antikörper während des Markierungsverfahrens aggregieren und mit Rheumafaktoren unspezifische Bindungen eingehen, die in einem Testverfahren mit markierten Antikörpern falsch positive Werte ergeben.

Die Erfindung hat sich die Aufgabe gestellt, unspezifische Antikörper-Antigen Bindungen auszuschließen. Dies wird dadurch erreicht, daß man für den Nachweis der Antikörper keine markierten Antikörper, sondern enzymmarkierte Erreger oder enzymmarkierte Spaltstücke von Erregern verwendet und wie in der DE—AS 27 33 380 schon beschrieben den Nachweis unter Verwendung eines festen Trägers durchführt, der mit einem Bindeprotein (Antikörper) beschichtet ist, der die nachzuweisenden spezifischen Immunglobulinklassen selektiv bindet. Beim Aufbringen einer Serumprobe, die auf vorhandene Antikörper untersucht werden soll, wird nur die nachzuweisende Immunglobulinklasse (IgM, IgA, IgG, IgE) gebunden. Andere Serumbestandteile, wie unerwünschte Immunglobuline und Albumin werden durch sorgfältiges Waschen entfernt. In der Serumprobe enthaltenes unerwünschtes menschliches IgG kann weder mit Rheumafaktoren reagieren noch Antigen blockieren, das zum Nachweis von IgM-Antikörpern verwendet wird. Die Markierung mit Enzymen schließt die Gefahren aus, die eine Markierung mit Radioisotopen zwangsläufig in sich birgt. Besondere Vorsichtsmaßnahmen sind ebensowenig notwendig wie kostspielige und komplizierte Vorrichtungen, die nur von besonders geschultem Personal bedient werden können. Die Enzymreaktionsprodukte lassen sich in jedem herkömmlich ausgestatteten Labor feststellen.

Die Markierung von Antikörpern nit Enzymen ist, wie oben schon erwähnt, von Nakane und Mitarbeitern in The Journal of Histochemistry and Cytochemistry, Band 22, Nr. 12, Seiten 1084—1091 (1974) beschrieben. Hier ist auch ausgeführt, daß die angegebene Methode generell zur Markierung von Proteinen, einschließlich proteinhaltiger Antigene geeignet sein müßte. Tatsächlich ist jedoch bis heute kein Verfahren bekanntgeworden, das Immunreaktionen unter Verwendung von enzymmarkierten Erregern oder Spaltstücken von Erregern in genauer und zuverlässiger Weise ermögliche und sich so für die Labordiagnose eignet. Entsprechend weisen Avrameas und Mitarbeiter in einer zusammenfassenden Arbeit in Scand. J. Immunol., Bd. 8, Ergänzung 7, Seite 7 ff. (1978) darauf hin, daß die meisten Markierungsverfahren die Kupplung von Enzymen an Antikörper betreffen und dennoch nicht alle dieser Verfahren für Enzymimmuntests geeignet sind. Hier ist auch auf die Schwierigkeiten einer effizienten Enzymkupplung unter Beibehaltung der Enzymwirksamkeit hingewiesen und ausgeführt, daß die Bindung von Enzymen an kleine, als Haptene bezeichnete organische Verbindungen ein anderes Verfahren darstellt. Ein Verfahren zur Markierung von Haptenen ist z.B. in der US—PS 3 839 153 beschrieben. Mikrobielle Erreger sind jedoch wesentlich labiler als Antikörper oder die als Haptene bezeichneten definierten organischen Verbindungen und erfordern zur Erzielung einer effizienten Markierung eine schonende Behandlung. Offensichtlich beruht dies auf den im Vergleich zu radioaktiv markierten Antigenen stärkeren Wechselwirkungen der enzymmarkierten Antigene mit den Bestandteilen der physiologischen Testflüssigkeit.

Überraschenderweise wurde jetzt gefunden, daß man eine äußerst effektive Markierung von mikrobiellen Antigenen unter wirksamer Unterdrückung von Hintergrundreaktionen erreicht, wenn man Peroxidase bei einem alkalischen pH-Wert von 9,3 in Gegenwart einer solchen Menge einer oder mehrerer alkalischer Puffersubstanzen an mikrobielle Erregerbinder, daß auch nach der Verdünnung des Enzymkonjugats und dessen Reaktion mit dem nachzuweisenden Antikörper ein alkalischer pH-Wert von 8 und darüber, z.B. von 8,7 aufrechterhalten wird. Falls notwendig, kann dieser alkalische pH-Wert durch weitere Zugabe eines alkalischen Materials eingestellt werden. Die äußerst effektive Markierung ermöglicht, daß selbst ein verhältnismäßig unreines mikrobielles Antigen markiert werden kann, ferner, daß das markierte Antigen in einer sehr hohen Verdünnung von bis zu 1:400 verwendet werden kann.

In seinen Einzelschritten besteht der erfindungsgemäße Immuntest darin, daß man

a) einen festen Träger mit Antikörpern beschichtet, die selektiv in einer Serumprobe enthaltene IgM-, IgA-, IgG- oder IgE-Antikörper zu binden vermögen,
b) den beschichteten Träger mit der Serumprobe in Kontakt bringt
c) die nicht gebundenen Komponenten der Serumprobe wegwäscht
d) die von dem beschichteten Träger selektiv gebundenen Antikörper bei einem H-Wert von 8 oder darüber mit einer Lösung in Berührung bringt, die alkalische Puffer, Kälberserum, nicht-ionisches Detergens und ein Peroxidasekonjugat enthält, das durch Kupplung eines mikrobiellen Erregers, der für den selektiv gebundenen Antikörper spezifisch ist, an Perioxidase in Gegenwart eines Puffers bei pH 9,3 erhalten wurde,
e) die nicht gebundenen Bestandteile der Lösung wegwäscht und
f) die Enzymwirksamkeit des Bindungsproduktes bestimmt.

Die Erfindung umfaßt auch einen Testsatz für die Durchführung des erfingungsgemäßen Enzymimmuntests, der die folgenden Komponenten umfaßt:

a) einen festen Träger, der mit Antikörpern beschichtet ist, die selektiv IgM-, IgA-, IgG- oder IgE-Antikörper zu binden vermögen
b) mit Peroxidase markierte mikrobielle Erreger, die für die selektiv gebundenen Antikörper spezifisch sind, in einer Puffersubstanz, Kälberserum und nichtionisches Detergens enthaltenden Lösung vom pH 8 oder darüber, wobei die markierten Erreger durch Kupplung an Peroxidase in Gegenwart eines Puffers bei pH 9,3 erhalten wurden, und
c) ein Substrat für die Enzymreaktion.

Die Erfindung stellt ein hochempfindliches Verfahren für die Labordiagnose zur Verfügung. Die bisher notwendige Reinigung des Enzymkonjugats durch Säulenchromatographie zur Entfernung nichtumgesetzter Substanzen ist ebenso überflüssig wie eine Blockierung freier $\varepsilon$- und $\alpha$-Aminogruppen im Proteinanteil zur Verhinderung ungewünschter Reaktionen. Die effektive Markierung sowie der glatte Verlauf der Bindungsreaktion zwischen dem markierten Erreger und dem nachzuweisenden Antikörper ist überraschend, da die Bedingungen, unter denen diese Bindungsreaktion durchgeführt wird, unphysiologisch sind und damit für den Fachmann nicht naheliegend waren. Weiterhin lassen sich unspezifische Bindungen des markierten mikrobiellen Antigens an die feste Phase erheblich reduzieren, wenn das markierte Antigen in einer Verdünnung angewandt wird, die Kälberserum, insbesondere Serum neugeborener Kälber und ein nichtionisches Detergens enthält, z.B. Polyoxyethylensorbitanmonolaurat (Tween 20). Das nichtionische Detergens bewirkt, daß nichtgebundenes Material durch Waschen leichter entfernt wird. Das in der Verdünnung des Enzymkonjugats enthaltene Kälberserum und das nichtionische Detergens halten unerwünschte Hintergrundreaktionen auf einem Minimum.

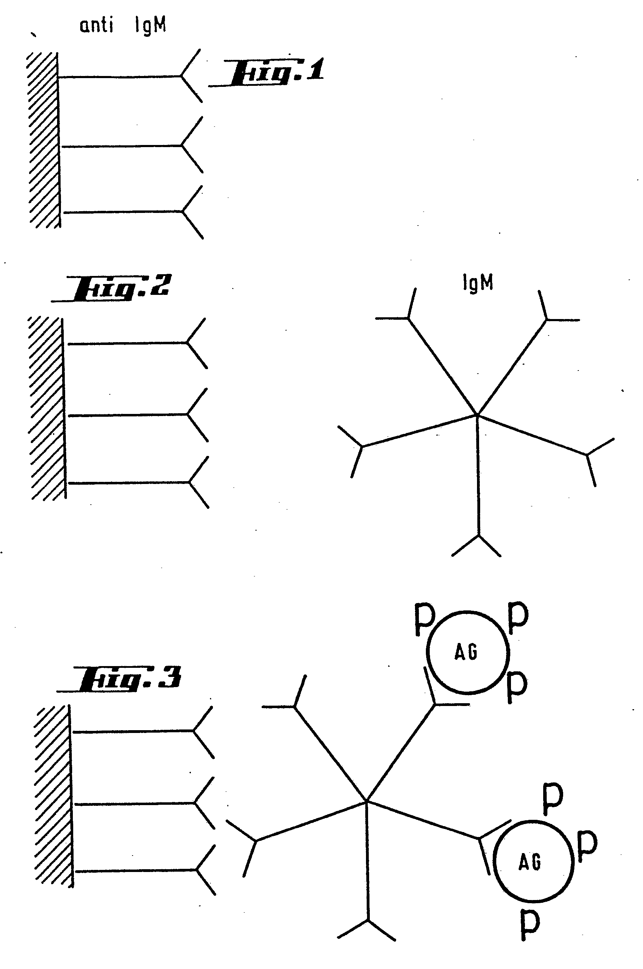

Das erfindungsgemäße Testverfahren ist schematisch in der beigefügten Abbildung wiedergegeben. In ihr zeigt

1. den mit spezifischem anti-Antikörper, nämlich anti-IgM beschichteten festen Träger,
2. den spezifischen, in der Serumprobe nachzuweisenden Antikörper IgM, der vom beschichteten festen Träger selektiv gebunden wird,
3. die erfolgte Bindungsreaktion zwischen anti-Antikörper, nachzuweisendem Antikörper und Peroxidasemarkiertem mikrobiellen Erreger=-anti-IgM:IgM:Ag (Peroxidase).

Für den Nachweis der Antikörper werden vorteilhaft mikrobielle Erreger oder Erregerspaltstücke verwendet, die von Faktoren befreit sind, die zu unerwünschten Antikörper-Reaktionen führen können, wie nukleären Bestandteilen.

Das erfindungsgemäße Verfahren ist aufgrund der effektiven Markierung und der Ausschaltung nicht spezifischer Antikörper-Antigen Bindungen hochempfindlich und außerordentlich spezifisch. Die zu bestimmenden spezifischen Antikörper lassen sich in einer Verdünnung von bis 1:1 000 000 nachweisen, obgleich negative Seren in einer Verdunnung von nur 1:100 keine Reaktion ergeben. Ein weiterer Vorteil des Erfindungsgemäßen Testverfahrens besteht darin, daß es gegenüber den bekannten, mit markierten Antikörpern arbeitenden Verfahren keine drei, sondern nur zwei Stufen erfordert, also einfacher und schneller durchzuführen ist.

Geeignete feste Träger für Immunglobulinspezifische Bindeproteine sind bekannt. Es können hierfür Cellulose-verbindungen, Dextrane und synthetische Polymere, wie Polystyrol und Polyvinylchlorid, in Form von Teilchen unterschiedlicher Größe, wie Körnern, Platten, Stäben oder Streifen verwendet werden. Die Bindeproteine können unter Anwendung bekannter physikalischer oder chemischer Methoden an diese Träger gebunden werden.

Das folgende Beispiel erläutert eine ausführungsform des erfindungsgemäßen Verfahrens. Selbstverständlich sind Modifizierungen im Rahmen der durch die Erfindung vermittelten Lehre unter Zuhilfenahme der dem Fachmann zur Verfügung stehenden Kenntnisse möglich.

Beispiel

Herstellung von gereinigtem enzymmarkierten mikrobiellen Antigen

A. Gewinnung von gereinigtem mikrobiellen Antigen

Für die Markierung viraler Antigene werden virusinfizierte (Gewebekultur) Zellen bei 3000 UpM 5 Minuten sedimentiert. Der Überstand wird abgegossen, und die Zellen werden einmal mit PBS (phosphatgepufferte Kochsalzlösung) gewaschen. Dann wird das Sediment in 1 ml destilliertem Wasser und 1 % nichtionischer Detergenslösung (NP40) aufgenommen und zur Aufschließung 10 mal je 3 Sekunden im Eisbad beschallt (Branson Sonifier, output control 7, microtip). Danach wird zur Entfernung grober Zellbestandteile 5 Minuten bei 3000 UpM abzentrifugiert. Der Überstand wird auf ein Kissen von 30 % Dextran-T10 (Pharmacia) in TE-Puffer (0,01 M TRIS (Tris-(hydrooxyethyl)aminoethan)-HCL+0,001 M EDTA (Ethylendiamintetraessigsäure), pH 7,4, geschichtet (Überstand/Dextran=1:9) und 1 bis 8 Stunden bei 35.000—60 000 UpM je nach viralem Antigen sedimentiert (Beckman Zentrifuge, SW 41). Das Sediment wird in 1 ml destilliertem Wasser aufgenommen und nochmals 3 mal je 5 Sekunden im Eisbad beschallt (Bedingungen wie oben). Danach wird der Proteingehalt nach der Methode von LOWRY bestimmt. Er soll bei 0,5 bis 1 mg/ml liegen.

B. Enzymmarkierung des mikrobiellen Antigens

1) Aktivierung der Peroxidase:

4 mg Meerrettich-Peroxidase (Sigma) werden in 1 ml destilliertem Wasser aufgenommen und mit 0,2 ml 1 M $NaJO_4$ (21 mg/ml destilliertes Wasser) versetzt. Die Lösung wird 20 Minuten bei 20°C inkubiert und soll sich dabei olivgrün färben. Danach wird über Nacht gegen 0,001 M Natrium-Acetat Puffer vom pH 4,4 dialysiert.

2) Kupplung der Peroxidase an des mikrobielle Antigen:

1 ml gereinigtes Virus mit einem Proteingehalt von 0,5—1 mg/ml wird nach Zugabe von 0,1 ml Natriumcarbonatpuffer (0,5 bis 1 M; pH 9,3) sofort mit der aktivierten Peroxidase im Gewichtsverhältnis 2 mg Peroxidase/1 mg Virusprotein versetzt. Anschließend wird 2,5 Stunden bei 4°C inkubiert. Nach dieser Zeit wird eine frisch angesetzt Lösung von 4 mg/ml $NaBH_4$ im Verhältnis 1/20 (V/V) zugegeben und erneut 2 Stunden bei 4°C inkubiert. Das fertige Konjugat wird in verschiedenen Verdünnungen in PBS+5 % Kälberserum gegen ein Serum mit und ein Serum ohne spezifische IgM-Antikörper (Serumverdünnung 1:100) ausgetestet. Die optimale Antigenverdünnung kann dann bei —70°C eingefroren oder mit 5 % Kälberserum lyophilisiert werden.

Bisher wurden humanes Cytomegalo-Virus, verschiedene andere Herpes Viren, Röteln Virus und Hepatitis A-Virus erfolgreich mit Peroxidas markiert.

Herstellung des mit anti-IgM für die selektiv zu bindenden Antikörper beschichteten Trägers

Als feste Phase oder fester Träger dienen flexible PVC-Mikrotiterplatten (Dynatech) mit 96 Flachbodenlöchern. Verdünnungen von gereinigten Kaninchen-Antikörpern, die für $\mu$-Ketten aber auch z.B. für $\alpha$-, $\gamma$- oder $\varepsilon$-Ketten von menschlichem Immunglobulin spezifisch sind (10 ml Protein/ml), werden im Verhältnis 1:300 in PBS hergestellt und mit 1 mg/ml $NaN_3$ verstetzt. Hiervon werden 0,1 ml in jedes Loch gefüllt, und die Platte wird bei 20°C in einer feuchten Kammer inkubiert. Nach 24 Stunden werden in jedes Loch 10 % Kälberserum, ebenfalls zusammen mit 1 mg/ml $NaN_3$ zugefügt, und es wird erneut 24 Stunden unter den gleichen Bedingungen inkubiert. Nach 10 Tagen bei 4°C können die Platten mit PBS gewaschen und nach einer letzten Waschbehandlung mit destilliertem Wasser getrocknet, mit einer Klarsichtfolie luftdicht verschlossen und bei 4°C mindestens sechs Monate aufbewahrt werden.

Ablauf des Bestimmungsverfahrens

Die beschichtete Platte wird nach Entfernung der Folie gewaschen. Dazu wird sie ausgeschüttelt und dann mit PBS, das 0,05 % Polyoxyethylensorbitanmonolaurat (Tween 20) enthält, gefüllt. Nach 60 Sek. wird die Platte ausgeleert. Dieser Vorgang wird 5mal wiederholt. Zum Schluß wird, zur Vermeidung von Blasen, zweimal kurz mit reinem PBS gewaschen. Dann wird die Platte über Kopf auf einer saugfähigen Unterlage ausgeschlagen. Sie darf jedoch keinesfalls austrocknen. Dieser Waschvorgang wird in gleicher Weise nach jedem Arbeitsgang wiederholt. Die Serumproben werden in PBS, das 1 mg/ml $NaN_3$ und zur besseren Sichtbarmachung eventuell 0,01 % Phenolrot enthält, verdünnt, in Löcher der beschichteten Platte getropft und zwei Stunden in einer feuchten Kammer bei 20°C inkubiert. Als Kontrolle laufen immer zwei positive und zwei negative Seren mit bekannten Antikörpertitern mit. Darauf wird gewaschen. Nun tropft man das lyophilisierte, in PBS und 0,5 % Polyoxyethylensorbitanlaurat (Tween 20) gelöste mikrobielle Antigen zu und inkubiert bei pH etwa 8,7 zwei Stunden bei 20°C in der feuchten Kammer. Ist die Platte nach diesem Schritt gewaschen, so kann gefärbt werden.

Färbung:

10 mg o-Phenylendiamin werden in 10 ml Phosphatpuffer (0,1 M, pH 6)+0,001 % $H_2O_2$ aufgelöst. Diese Lösung muß kurz vor dem Faben frisch angesetzt werden und darf keinem direkten Lichteinfall ausgesetzt werden. 50 $\mu l$ werden in jedes Loch gegeben. Nach 5—6 Minuten wird die Reaktion durch Zugabe von 0,1 ml 2 M $H_2SO_4$ gestoppt. Die Auswertung erfolgt mit bloßem Auge oder photometrisch bei 490 nm.

**Patentansprüche**

1. Enzymimmuntest unter Verwendung eines an einen festen Träger gebundenen ersten immunologischen Reaktionspartners, der mit einer einen zweiten immunologischen Reaktionspartner enthaltenden Lösung in Kontakt gebracht und nach Aufhebung des Kontaktes mit einem gegen den nachzuweisenden zweiten Reaktionspartner gerichteten markierten dritten immunologischen Reaktionspartner in Berührung gebracht wird, dadurch gekennzeichnet, daß man

a) einen festen Träger mit Antikörpern beschichtet, die selektiv in einer Serumprobe enthaltene IgM-, IgA-, IgG- oder IgE-Antikörper zu binden vermögen,
b) den beschichteten Träger mit der Serumprobe in Kontakt bringt
c) die nicht gebundenen Komponenten der Serumprobe wegwäscht
d) die von dem beschichteten Träger selektiv gebundenen Antikörper bei einem pH-Wert von 8 oder darüber mit einer Lösung in Berührung bringt, die alkalische Puffer, Kälberserum, nicht-ionisches Detergens und ein Peroxidasekonjugat enthält, das durch Kupplung eines mikrobiellen Erregers, der für den selektiv gebundenen Antikörper spezifisch ist, an Peroxidase in Gegenwart eines Puffers bei pH 9,3 erhalten wurde
e) die nicht gebundenen Bestandteile der Lösung wegwäscht und
f) die Enzymwirksamkeit des Bindungsproduktes bestimmt.

2. Enzymimmuntest nach Anspruch 1, dadurch gekennzeichnet, daß man den Träger mit gereinigten Antikörpern beschichtet, die selektiv IgM- oder IgA-Antikörper binden.

3. Enzymimmuntest nach Anspruch 1 und 2, dadurch gekennzeichnet, daß die enzymmarkierten mikrobiellen Erreger im wesentlichen frei von nukleären Bestandteile sind.

4. Enzymimmuntest nach Anspruch 1 bis 3, dadurch gekennzeichnet, daß man die enzymmarkierten mikrobiellen Erreger in einer Verdünnung verwendet, die je ml etwa 1,5 mg Enzym und etwa 1 mg Erregerprotein enthält.

5. Enzymimmuntest nach Anspruch 1 bis 4, dadurch gekennzeichnet, daß der mikrobielle Erreger aus Cytomegalo-Virus besteht.

6. Testatz für die Durchführung des Enzymimmuntests nach Anspruch 1 bis 5 enthaltend

a) einen festen Träger, der mit Antikörpern beschichtet ist, die selektiv IgM-, IgA-, IgG- oder IgE-Antikörper zu binden vermögen
b) mit Peroxidase markierte mikrobielle Erreger, die für die selektiv gebundenen Antikörper spezifisch sind, in einer Puffersubstanz, Kälberserum und nicht-ionisches Detergens enthaltenden Lösung vom pH 8 oder darüber, wobei die markierten Erreger durch Kupplung an Perioxidase in Gegenwart eines Puffers bei pH 9,3 erhalten wurden, und
c) ein Substrat für die Enzymreaktion.

**Revendications**

1. Essai immunoenzymatique par l'utilisation d'un premier partenaire réactionnel immunologique qui est fixé sur un support solide, est amené en contact avec une solution contenant un deuxième partenaire réactionnel immunologique et, après suppression du contact, est amené en contact d'un troisième partenaire réactionnel immunologique marqué, dirigé vers le deuxième partenaire réactionnel à détecter, caractérisé en ce que

a) on enduit un support solide d'anticorps qui sont capables de fixer de manière sélective des anticorps IgM, IgA, IgG ou IgE contenus dans un échantillon de sérum,
b) on met le support enduit en contact avec l'échantillon de sérum,
c) on élimine par lavage les composants non fixés de l'échantillon de sérum,
d) à une valeur de pH de 8 ou davantage, on met les anticorps fixés de manière sélective par le support enduit en contact avec une solution qui contient un tampon alcalin, du sérum de veau, un détecgent non ionogène et un produit de conjugaison de peroxydase qui a été obtenu, à un pH de 9,3, par couplage d'un agent pathogène microbien, qui est spécifique pour les anticorps fixés de manière sélective, sur de la peroxydase, en présence d'un tampon,
e) on élimine par lavage les éléments non fixés de la solution, et

f) on détermine l'activité enzymatique du produit de la fixation.

2. Essai immunoenzymatique suivant la revendication 1, caractérisé en ce qu'on enduit le support d'anticorps purifiés qui fixent de manière sélective des anticorsp IgM ou IgA.

3. Essai immunoenzymatique suivant l'une des revendications 1 et 2, caractérisé en ce que les agents pathogènes microbiens marqués par une enzyme sont essentiellement exempts d'éléments nucléaires.

4. Essai immunoenzymatique suivant l'une des revendications 1 à 3, caractérisé en ce qu'on utilise les agents pathogènes microbiens marqués par une enzyme dans une dilution qui contient par ml environ 1,5 mg d'enzyme et environ 1 mg de protéine d'agent pathogène.

5. Essai immunoenzymatique suivant l'une des revendications 1 à 4, caractérisé en ce que l'agent pathogène microbien est constitué du virus cytomégalique.

6. Trousse d'essai pour la mise en oeuvre de l'essai immunoenzymatique suivant l'une des revendications 1 à 5, contenant

a) un support solide qui est enduit par des anticorps qui sont capables de fixer de manière sélective des anticorps IgM, IgA, IgG ou IgE,

b) des agents pathogènes microbiens marqués par de la peroxydase, qui sont spécifiques pour les anticorps fixés de manière sélective, dans une solution d'un pH de 8 ou davantage, qui contient une substance tampon, du sérum de veau et un détergent non ionogène, les agents pathogènes marqués ayant été obtenus à un pH de 9,3 par couplage sur de la peroxydase, en présence d'un tampon, et

c) un substrat pour la réaction enzymatique.

**Claims**

1. Enzyme immunoassay using a first immunological reactant which is bound to a solid support and which is contacted with a solution containing a second immunological reactant and after the removal of this contact is contacted with a labeled third reactant for the second reactant to be determined, characterized in

a) coating a solid support with antibodies which selectively bind IgM, IgA, IgG or IgE antibodies which are contained in a serum specimen

b) contacting the coated support with the serum specimen

c) removing unbound components of the specimen

d) reacting the antibodies which are selectively bound by the coated support at pH 8 or above with a solution containing alkaline buffers, calf serum, non-ionic detergent and a peroxidase conjugate which has been obtained by coupling a microbial antigen which is specific to the bound antibody to peroxidase at pH 9,3 in the presence of a buffer

e) removing the unbound components of the solution by washing

f) determining the enzyme activity of the reaction product.

2. Enzyme immunoassay according to claim 1, characterized in that the support is coated with purified antibodies which selectively bind IgM or IgA antibodies.

3. Enzyme immunoassay according to claims 1 and 2, characterized in that the used enzyme-labelled antigen is substantially free from nucleic materials.

4. Enzyme immunoassay according to claims 1 to 3, characterized in that the enzyme labelled microbial antigens are used in a dilution which contains per ml about 1,5 mg enzyme and about 1 mg microbial protein.

5. Enzyme immunoassay according to claims 1 to 4, characterized in that the microbial antigen is Cytomegalo virus.

6. Test kit for performing the enzyme immunoassay according to claims 1 to 5, comprising

a) a solid support which is coated with antibodies which selectively bind IgM, IgA, IgG or IgE antibodies

b) peroxidase-labeled microbial antigens which are specific to the selectively bound antibodies in a solution of pH 8 or above which contains buffer, calf serum and non-ionic detergent, the labelled antigen having been obtained by coupling to peroxidase in the presence of a buffer at pH 9,3, and

c) a substrate for the enzyme reaction.

0 023 989

anti IgM

**Fig.1**

**Fig.2**

IgM

**Fig.3**

AG

AG